# EUROPEAN PATENT APPLICATION

(11) **EP 0 768 093 A1**
(43) Date of publication of application: **16.04.1997**
(21) Application number: 96116057.9
(22) Date of filing: 08.10.1996
(51) Int. Cl.: A61M 5/315

(54) **Plunger stopper for syringe barrels**

(30) Priority: 13.10.1995 DE 29516282 U
(71) Applicant: HOECHST AKTIENGESELLSCHAFT, 65929 Frankfurt am Main (DE)
(72) Inventor: Koch, Hermann, 55130 Mainz (DE); Loos, Hans-Joachim, 65462 Ginsheim-Gustavsburg (DE)

(57) **Abstract**

The invention relates to a plunger stopper (5) for syringe barrels (6), the plunger stopper having one or more constrictions (4) and a first and a second end, wherein at least the first end of the plunger stopper (5) is of tapered design (2). In a first particular embodiment, at least the first end is of conically tapered design. In a further particular embodiment, at least one end is provided with a bore (3). However, both ends can also be of conically tapered design and can be provided with a bore.

## Description

The invention relates to a plunger stopper for syringe barrels, the plunger stopper having one or more constrictions and a first and a second end.

Plunger stoppers of the said type are known and are used for the closure and for the application of liquid pharmaceutical preparations in or from syringe barrels, one end of the plunger stopper pointing outward, if appropriate with a bore. Such a bore serves to produce a non-positive connection to a syringe frame. The contact area between the plunger stopper and the syringe barrel is reduced by the constrictions. This reduces the friction during the movement of the plunger stopper in the barrel. If syringe barrels which are closed with a plunger stopper of this type are to be subjected to sterilization using steam, the plunger stopper displays the following disadvantageous behavior: as a result of the thermal expansion of the syringe barrel and of the enclosed liquid, part of the plunger stopper is pushed out of the syringe barrel. In this case, one of the constrictions comes into contact with the steam. During cooling, steam condenses in the constriction and, as a result of the reverse movement of the plunger stopper due to cooling, is also drawn as condensate into the syringe barrel. This may cause the user to assume that the syringe barrel is leaking and that there is the risk of infection. This may lead to complaints and thus cause additional costs.

The invention is intended to provide an improved plunger stopper which overcomes the deficiencies of the prior art plunger stoppers.

An object of the invention is to provide an improved plunger stopper which can move easily in the syringe barrel yet maintain a seal with the walls of the barrel to prevent any fluid leakage.

Another object of the invention is to provide a plunger stopper that prevents condensation water from being drawn into the syringe barrel during steam sterilization.

The foregoing and other objects and advantages of the invention will be set forth in or are apparent from the following description.

The objects of the invention are carried out by means of a plunger stopper, wherein at least the first end of the plunger stopper is of tapered design.

The subject-matter of the invention is thus a plunger stopper for syringe barrels, the plunger stopper having one or more constrictions and a first and a second end, wherein at least the first end of the plunger stopper is of tapered design.

In a first particular embodiment, at least the first end is of conically tapered design. In a further particular embodiment, at least one end is provided with a bore. However, both ends can also be of conically tapered design and can be provided with a bore.

The advantage of the plunger stopper according to the invention is to be seen essentially in the fact that, during steam sterilization, no condensation water is drawn into the syringe barrel.

The plunger stopper can be designed as a single-hole or double-hole plunger stopper or as a solid stopper with constrictions of equal or different sizes arranged symmetrically or asymmetrically. The constrictions can be shaped to be cylindrical, trapezoidal, fully rounded or of similar geometries.

The plunger stopper according to the invention is described in greater detail with reference to Figures 1 to 4.

Fig. 1 illustrates a syringe barrel with an asymmetrical single-hole plunger stopper having two constrictions, one end of the plunger stopper containing a bore (hole) and a conically tapered design.

Fig. 2 illustrates a plunger stopper in a syringe barrel during steam sterilization.

Fig. 3 illustrates a symmetrical double-hole plunger stopper having two constrictions, both ends of the plunger stopper having a hole and a conically tapered design.

Fig. 4 illustrates a symmetrical double-hole plunger stopper having three constrictions, both ends having a hole and a conically tapered design.

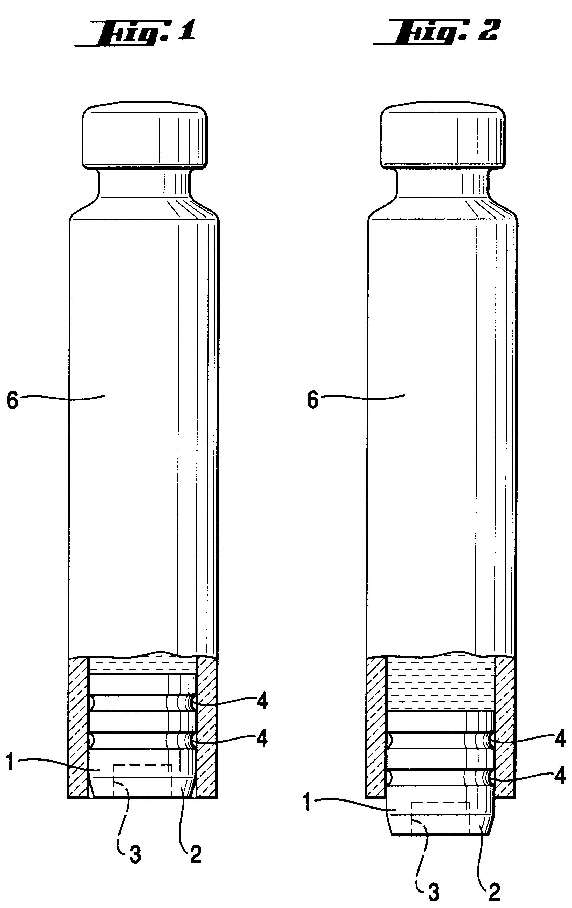

Figure 1 shows a syringe barrel (6) with an asymmetrical single-hole plunger stopper (1) having two constrictions (4) in the body of the plunger stopper. The constrictions reduce friction during the movement of the plunger stopper in the syringe barrel. The syringe barrel has an inlet end portion (front end) which generally contains an orifice through which the liquid is ejected, and an outlet end portion (back end). The plunger stopper is provided with a first end facing the outlet end portion of the syringe barrel and a second end facing the inlet end portion of the syringe barrel. The second end of the plunger stopper in Figure 1 is provided with a conically tapered design (2) and a hole (3). The hole or bore serves to produce a non-positive connection to the syringe frame.

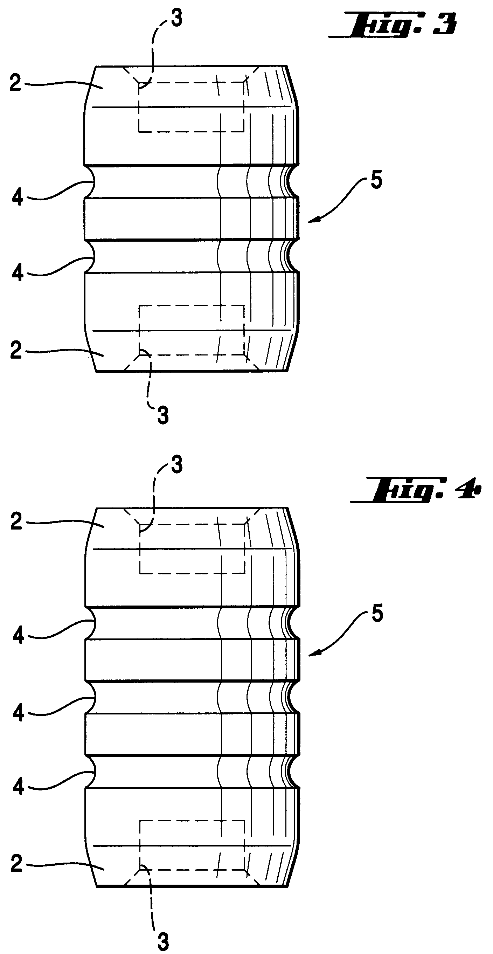

When the syringe barrels containing the plunger stoppers of Figure 1 are subjected to steam sterilization, part of the plunger stopper is pushed out of the syringe barrel as shown in Figure 2. This figure illustrates that the constrictions do not come in contact with the steam from the sterilization procedure and therefore do not draw water into the syringe barrel.

Figure 3 shows a symmetrical double-hole plunger stopper (5) having two constrictions (4). The contact area between the plunger stopper and the syringe barrel is reduced by the constrictions so that less friction is generated during the movement of the plunger stopper in the barrel. In this figure, both ends of the plunger stopper have a conically tapered design (2) and both ends contain a hole or a bore (3) to provide a non-positive connection to the syringe frame.

Figure 4 is similar to Figure 3 except that the symmetrical double-hole plunger stopper (5) has three constrictions (4) instead of two. In this figure, both ends of the plunger stopper have a conically tapered design (2) and both ends contain a hole or a bore (3).

The above description of the invention is intended to be illustrative and not limiting. Various changes or modifications in the embodiments described may occur to those skilled in the art. These can be made without departing from the spirit or scope of the invention.

## Claims

1. A plunger stopper for a syringe barrel, the plunger stopper having at least one constriction and a first end and a second end, wherein the first end of the plunger stopper is tapered.

2. The plunger stopper as claimed in claim 1, wherein the second end of the plunger stopper is tapered.

3. The plunger stopper as claimed in claim 1, wherein the first end of the plunger stopper in conically tapered.

4. The plunger stopper as claimed in claim 3, wherein the second end of the plunger stopper is conically tapered.

5. The plunger stopper as claimed in claim 1, wherein the first end of the plunger stopper has a bore.

6. The plunger stopper as claimed in claim 1, wherein the second end of the plunger stopper has a bore.

7. A syringe which comprises:
a syringe barrel having an inlet end portion and an outlet end portion; and a plunger stopper, slidably disposed within the syringe barrel, the plunger stopper having at least one constriction, a first end facing the outlet end portion of the syringe barrel, and a second end facing the inlet end portion of the syringe barrel, wherein the first end of the plunger stopper is tapered.

8. A syringe which comprises:
a syringe barrel having an inlet end portion and an outlet end portion; and a plunger stopper, slidably disposed within the syringe barrel, the plunger stopper having at least one constriction, a first end facing the outlet end portion of the syringe barrel, and a second end facing the inlet end portion of the syringe barrel, wherein the second end of the plunger stopper is tapered.

9. The syringe as claimed in claim 7, wherein the second end of the plunger stopper is tapered.

10. The syringe as claimed in claim 7, wherein the first end of the plunger stopper has a bore.
